# EUROPEAN PATENT APPLICATION

(11) **EP 2 324 757 A1**
(43) Date of publication of application: **25.05.2011**
(21) Application number: 10251955.0
(22) Date of filing: 18.11.2010
(51) Int. Cl.: A61B 1/32

(54) **Access device including an integrated light source**

(30) Priority: 19.11.2009 US 262642 P; 29.10.2010 US 915108
(71) Applicant: Tyco Healthcare Group LP, New Haven, CT 06511 (US)
(72) Inventor: Stopek, Joshua, Guilford, CT 06437 (US)
(74) Representative: Soames, Candida Jane

(57) **Abstract**

A seal anchor member including leading and trailing ends, wherein the leading end includes a light source. The light source may be powered by an internal or an external power source. The light source may include, but is not limited to, light emitting diodes and/or fiber optic cables. The light source may emit light having any known wavelength, e.g., visible, ultraviolet, and near-infrared light.

## Description

### CROSS REFERENCE TO RELATED APPLICATIONS

This application claims priority to, and benefit of, U.S. Provisional Application Serial Number 61/262,642, filed November 19, 2009, the disclosure of which is hereby incorporated by reference in its entirety.

### BACKGROUND

### 1. Technical Field

The present disclosure relates to medical devices including an integrated light source. More particularly, the present disclosure relates to an access device including an integrated light source.

### 2. Background of Related Art

Minimally invasive procedures are procedures that are performed through small incisions in a patient's skin. Such procedures have several advantages over traditional, open surgeries, such as reduced trauma to the patient and a faster recovery time. Generally, such procedures are referred to as "endoscopic", unless performed on the patient's abdomen. Procedures that are performed on the abdomen are referred to as "laparoscopic". Throughout the present disclosure, the term "minimally invasive" should be understood to encompass both endoscopic and laparoscopic procedures.

During a typical minimally invasive procedure, surgical objects, such as surgical access devices (e.g., trocar and cannula assemblies) or endoscopes, are inserted into the patient's body through an incision in tissue or into a naturally occurring orifice (*e.g*., mouth, anus, or vagina). In general, prior to the introduction of the surgical object into the patient's body, insufflation gases are used to enlarge the area surrounding the target surgical site to create a larger, more accessible work area.

Minimally invasive procedures are complicated by a number of visual limitations. In particular, the surgeon's view is limited to what is directly in front of and is illuminated by a scope inserted in the incision. To inhibit damage to internal body structures, it is desirable to illuminate the surgeon's viewing field to a greater degree than is currently possible.

### SUMMARY

Disclosed herein is a seal anchor member that is configured and adapted for placement within a tissue tract. The seal anchor member includes a housing that includes leading and trailing ends. One or both of the trailing and leading ends may define surfaces that are substantially convex or concave to assist in the insertion of seal anchor member the within tissue tract. An intermediate portion is positioned between the leading and trailing ends and is adapted to be positioned within the tissue tract in a substantially sealed relation with tissue surfaces that define the tissue tract. At least one longitudinal port extends between the leading and trailing ends, and is adapted to receive an instrument therein in a substantially sealed relation.

The seal anchor member may be formed from a compressible material that is adapted to transition between a first expanded condition and a second compressed condition. The first expanded condition facilitates securing of the seal anchor member within the tissue tract. The second compressed condition facilitates at least partial insertion of the seal anchor member within the tissue tract.

At least one light source is operatively associated with the leading end. The light source may be operatively coupled to either an external or an internal power source, *e.g*., a battery. The at least one light source may be a light emitting diode (LED) or may be a fiber optic cable that is operatively coupled to an external light source. Other light sources as are known in the art may be used as well. The light source may be selectively positionable, *i.e*.., reoriented with respect to the leading end, to facilitate the illumination of particular areas of interest within the body cavity.

The light source may emit light having any known wavelength. For example, the light source may emit visible, ultraviolet, or near-infrared light. In an embodiment, the light source may emit light adapted to polymerize a sealant or an adhesive. In another embodiment, the light source may emit light adapted to excite a biomarker or an immunological agent or dye. In addition, in certain applications, the light sources may emit more than one type, *i.e,* wavelength of light. Moreover, the intensity of the light emitted from the light sources may be adjusted before or during a procedure, *i.e*., decreased or increased as desired, or otherwise regulated. In addition, the intensity and wavelength, as well as the activation, of the light sources may be synchronized to the use or activation of other instruments, *e.g*., camera or light detection systems.

These and other features of the apparatus disclosed herein will become more readily apparent to those skilled in the art from the following detailed description of various embodiments of the present disclosure.

### BRIEF DESCRIPTION OF THE DRAWINGS

Various embodiments of the present disclosure are described hereinbelow with reference to the drawings, wherein:

Fig. 1 is a front perspective view of an embodiment of a seal anchor member in accordance with the present disclosure shown positioned within tissue;

Fig. 2 is a bottom view of the seal anchor member of Fig. 1; and

Fig. 3 is a front perspective view of an access system including seal anchor member and a light source in accordance with the present disclosure.

### DETAILED DESCRIPTION OF EMBODIMENTS

In the drawings and in the description which follows, in which like references numerals identify similar or identical elements, the term "proximal" will refer to the end of the apparatus which is closest to the clinician during use, while the term "distal" will refer to the end which is furthest from the clinician, as is traditional and known in the art.

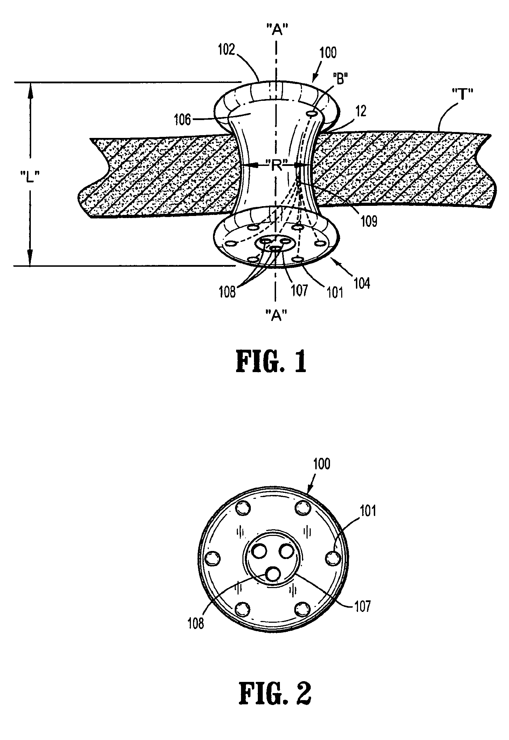

A seal anchor member 100 will now be described with reference to Figs. 1-2. Seal anchor member 100 is configured and adapted to be inserted within a tissue tract 12 defined by tissue surfaces formed in tissue "T", e.g., an incision. Although the presently described seal anchor member 100 is discussed in connection with minimally invasive procedures, it is within the scope of the present disclosure that the seal anchor member may be used through a naturally occurring opening (e.g., anus or vagina) or any incision in a patient's skin.

Seal anchor member 100 defines a longitudinal axis "A" and has respective trailing (proximal) and leading (distal) ends 102, 104. Intermediate portion 106 is disposed between the trailing and leading ends 102, 104. As depicted in Fig. 1, trailing and leading ends 102, 104 define substantially planar surfaces. However, in other embodiments, either or both of the trailing and leading ends 102, 104 define surfaces that are substantially convex or concave to assist in the insertion of seal anchor member 100 within tissue tract 12. A working chamber 107 may include one or more lumens 108 that are longitudinally disposed between the trailing and leading ends 102, 104 and are adapted to receive a surgical instrument in a substantially sealed relation.

Intermediate portion 106 defines a radial dimension "R" and extends longitudinally between the trailing and leading ends 102, 104 to define an axial dimension or length "L". As shown in Fig. 1, the radial dimension "R" of intermediate portion 106 varies along the length "L", i.e., the cross-sectional dimension may vary along length "L" and may have an hour-glass configuration. The hour-glass configuration of the intermediate portion 106 facilitates anchoring of the seal anchor member 100 within tissue tract 12 defined by tissue surfaces formed in tissue "T". However, in other embodiments, the radial dimension "R" may remain substantially uniform along the length "L".

The seal anchor members 100 may be formed from a compressible material and may be adapted to transition between a first expanded condition to facilitate securing of the seal anchor member 100 within the tissue tract 12 and in a substantial sealed relation with tissue surfaces "T" defining the tissue tract 12, and a second compressed condition to facilitate at least partial insertion of the seal anchor member 100 within the tissue tract 12.

As shown in Figs. 1 and 2, one or more light sources 101 may be disposed on the distal end 104 of the seal anchor member 100. Light sources 101 may be chosen from any known light source, including but not limited to, light emitting diodes (LEDs) and incandescent lights.

As shown in Fig. 2, the light sources 101 are positioned within the leading end 104. The one or more light sources 101 may be selectively positioned with respect to the leading end 104. As such, one or more of the light sources 101 may be rotated or axially repositioned with respect to the distal end 104 of the seal anchor member. It is also contemplated that one or more of the light sources 101 may be radially repositionable with respect to the seal anchor member 100. In this configuration, the light source 101 provides a larger illumination field thereby increasing the illumination of the working space beyond the distal end 104 of the seal anchor member 100. By adjusting the orientation of the light sources 101 with respect the leading end 104, the illumination of particular internal body structures is facilitated.

An internal battery "B", shown in Fig. 1, may power the light sources 101 and may be operatively coupled to the light sources through power leads 109. In other embodiments, each of the light sources 101 may have its own power source. In still other embodiments, the light sources 101 may be powered by a power source that is external to the seal anchor member 100.

Light source 101 may be selected to have a desired wavelength depending on its intended use. For example, some procedures may involve the use of light sensitive materials that are responsive to certain wavelengths of light and the light source 101 may be selected accordingly. Some procedures may involve the application of light sensitive sealants and/or adhesives. For example, photocurable surgical tissue adhesives that are composed of photoreactive gelatin and poly (ethylene glycol) diacrylate may be used in laparoscopic procedures to effect prompt and effective hemostasis. For such procedures, light sources 101 may emit light of any known wavelength of light including, but not limited to, ultraviolet, visible, and near-infrared light.

The light sources 101 are integrated into the seal anchor member 100 and may emit light having the same, different, or variable wavelengths depending on the application. Therefore, two or more light sources 101 having different wavelengths and/or properties may be included within a single seal anchor member 100. For example, one light source 101 may be adapted to emit visible light, e.g., white light, for traditional illumination, while other light sources 101 may be adapted to excite biomarkers or immunological agents/dyes adapted to mark certain structures, e.g., vasculature, lymph, nerve, and neural networks, or conditions, e.g., tumors or other diseases.

During a procedure, a clinician may activate, deactivate, and regulate the light emitted from light sources 101. For example, the intensity of the light emitted from the light sources 101 may be adjusted during the procedure. Some procedures may include the application of biologically active compounds that are light-responsive. By adjusting the light exposure, the concentration of the active form of such compounds may be controlled. Some molecules can either be can either be irreversibly activated with light or reversibly switched between active and inactive states.

In a further embodiment, the light sources 101 may be adapted to be synchronized with other instruments such as camera and/or light detection systems (not shown) to provide on demand direct local illumination and visualization as needed. For example, operation of the camera may be synchronized with the emission of light into the body space on which the procedure is being performed. Wireless or other means may be used to synchronize the light sources 101 with the other instruments.

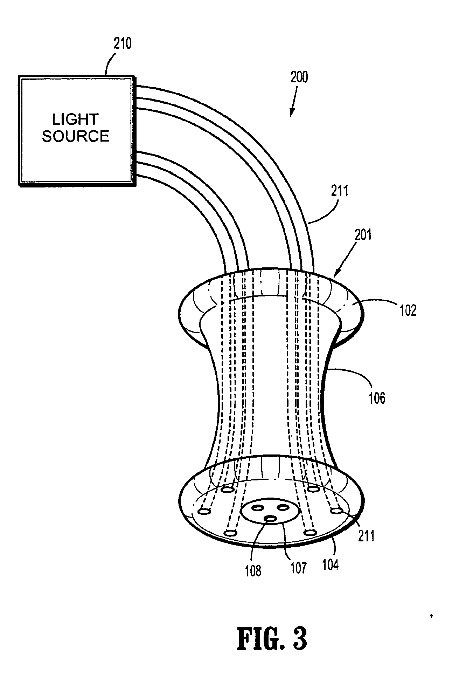

In yet another embodiment, as illustrated in Fig. 3, an access system 200 includes a seal anchor member 201, a light source 210, and fiber optic cables 211. The seal anchor member 201 is substantially similar to the seal anchor member 100 described above, and is adapted to be inserted within a tissue tract in a substantially identical manner as described above with reference to seal anchor member 100.

The differences between the seal anchor members 100, 201 will now be described. In particular, the seal anchor member 201 includes trailing and leading ends 102, 104, an intermediate portion 106, a working chamber 107 may include one or more lumens 108, and one or more fiber optics disposed within the seal anchor member 201 operatively coupled to an external light source 210 and terminating in the leading end 104.

Although light source 210 is shown external to the seal anchor member 201, in other embodiments the light source to which the fiber optic cables 211 are connected may be integral to the seal anchor member 201. Each of the fiber optics 211 may be adapted to emit light of a wavelength suitable for its intended use.

Although the illustrative embodiments of the present disclosure have been described herein with reference to the accompanying drawings, the above description, disclosure, and figures should not be construed as limiting, but merely as exemplifications of particular embodiments. It is to be understood that the disclosure is not limited to those precise embodiments, and that various other changes and modifications may be effected therein by one skilled in the art without departing from the scope or spirit of the disclosure.

The invention may be described by reference to the following numbered paragraphs:-
1. A seal anchor member comprising a housing including leading and trailing ends; an intermediate portion positioned between the leading and trailing ends, the intermediate portion being adapted to be positioned within a tissue tract in a substantially sealed relation with tissue surfaces defining the tissue tract; at least one longitudinal port extending between the leading and trailing ends, the at least one longitudinal port being adapted to receive an instrument therein in a substantially sealed relation; and at least one light source operatively associated with the leading end.
2. The seal anchor member of paragraph 1, wherein the at least one light source is operatively coupled to a power source that is integrated into the housing.
3. The seal anchor member of paragraph 2, wherein the power source is a battery.
4. The seal anchor member of paragraph 1, wherein the at least one light source is operatively coupled to a power source that is external to the housing.
5. The seal anchor member of paragraph 1, wherein the at least one light source is a light emitting diode.
6. The seal anchor member of paragraph 1, wherein the at least one light source is a fiber optic cable that is operatively coupled to an external light source.
7. The seal anchor member of paragraph 1, wherein the at least one light source is selectively positionable.
8. The seal anchor member of paragraph 1, wherein the seal anchor is further comprised of a compressible material and is adapted to transition between a first expanded condition to facilitate securing of the seal anchor member within the tissue tract and second compressed condition to facilitate at least partial insertion of the seal anchor member within the tissue tract.
9. The seal anchor member of paragraph 1, wherein at least one of the leading and trailing ends has a concave configuration to facilitate insertion of the seal anchor member within the tissue tract.
10. The seal anchor member of paragraph 1, wherein the at least one light source emits light selected from the group consisting of: visible, ultraviolet, and near-infrared light.
11. The seal anchor member of paragraph 1, wherein during use of the seal anchor member, the wavelength of the light emitted from the light source is adjustable.
12. The seal anchor member of paragraph 1, wherein a plurality of light sources emit light having at least two different wavelengths.
13. The seal anchor member of paragraph 11, wherein intensity of the light emitted is adjustable.

## Claims

1. A seal anchor member comprising:
a housing including leading and trailing ends;
an intermediate portion positioned between the leading and trailing ends, the intermediate portion being adapted to be positioned within a tissue tract in a substantially sealed relation with tissue surfaces defining the tissue tract;
at least one longitudinal port extending between the leading and trailing ends, the at least one longitudinal port being adapted to receive an instrument therein in a substantially sealed relation; and
at least one light source operatively associated with the leading end; and
wherein the seal anchor is further comprised of a compressible material and is adapted to transition between a first expanded condition to facilitate securing of the seal anchor member within the tissue tract and second compressed condition to facilitate at least partial insertion of the seal anchor member within the tissue tract.

2. The seal anchor member of claim 1, wherein the at least one light source is operatively coupled to a power source that is integrated into the housing.

3. The seal anchor member of claim 2, wherein the power source is a battery.

4. The seal anchor member of claim 1, wherein the at least one light source is operatively coupled to a power source that is external to the housing.

5. The seal anchor member of any preceding claim, wherein the at least one light source is a light emitting diode.

6. The seal anchor member of any of claims 1 to 4, wherein the at least one light source is a fiber optic cable that is operatively coupled to an external light source.

7. The seal anchor member of any preceding claim, wherein the at least one light source is selectively positionable.

8. The seal anchor member of any preceding claim, wherein at least one of the leading and trailing ends has a concave configuration to facilitate insertion of the seal anchor member within the tissue tract.

9. The seal anchor member of any preceding claim, wherein the at least one light source emits light selected from the group consisting of: visible, ultraviolet, and near-infrared light.

10. The seal anchor member of any preceding claim, wherein during use of the seal anchor member, the wavelength of the light emitted from the light source is adjustable.

11. The seal anchor member of any preceding claim, wherein a plurality of light sources emit light having at least two different wavelengths.

12. The seal anchor member of claim 10, wherein intensity of the light emitted is adjustable.
